# FASCICULE DE BREVET EUROPEEN

(11) **EP 2 975 996 B1**
(45) Date de publication et mention de la délivrance du brevet: **21.02.2018**
(21) Numéro de dépôt: 14713544.6
(22) Date de dépôt: 06.03.2014
(51) Int. Cl.: A61B 3/113

(54) **EQUIPEMENT ET PROCEDE POUR LA DETERMINATION DE L' OEIL DIRECTEUR**
VORRICHTUNG UND VERFAHREN ZUR BESTIMMUNG DES DOMINANTEN AUGES
APPARATUS AND METHOD FOR DETERMINING THE DOMINANT EYE

(30) Priorité: 21.03.2013 FR 1352519
(43) Date de publication de la demande: 27.01.2016
(73) Titulaire: Essilor International, 94220 Charenton-le-Pont (FR)
(72) Inventeur: HADDADI, Ahmed, F-94220 Charenton-le-Pont (FR)
(74) Mandataire: Allain, Laurent
(86) Numéro de dépôt international: PCT/FR2014/050514
(87) Numéro de publication internationale: WO 2014/147317

(56) Documents cités:
- WO-A1-01/47463
- WO-A1-2006/106248
- FR-A1- 2 972 339
- FR-A1- 2 972 911

## Description

L'invention se rapporte à un équipement pour la détermination de l'oeil directeur. Cette invention concerne le domaine de la personnalisation des verres correcteurs, et porte plus spécifiquement sur un équipement apte à déterminer le paramètre de dominance oculaire encore appelé « oeil directeur », d'un porteur de lunettes. L'invention concerne également un procédé de mesure de cet oeil directeur au moyen dudit équipement.

De tels procédés et équipements existent. On peut par exemple citer le brevet FR2972339, qui décrit un procédé de détermination de l'oeil directeur d'un porteur de lunettes, au moyen d'un dispositif de visée muni d'une fenêtre optique, et d'un système d'acquisition d'images. Le principe d'un tel procédé repose sur la visualisation d'une cible par le porteur à travers ladite fenêtre optique, et sur l'acquisition d'une image montrant le visage dudit porteur et la position de la fenêtre optique au moment de cette visualisation. Un traitement de l'image obtenue, permet de déterminer sans ambigüité l'oeil directeur de l'individu. Le dispositif de visée est matérialisé par une tablette manuelle, équipée de marqueurs de repérage et d'une fenêtre optique. Ce procédé se déroule de façon ergonomique et pratique pour le porteur, puisque l'opération de visualisation de la cible s'effectue les deux yeux ouverts, ledit procédé étant solide et rigoureux car l'information fournie concernant l'oeil directeur, est indiscutable et parvient directement audit porteur sans que celui-ci n'ait à effectuer la moindre démarche. Toutefois, un inconvénient de ce type de procédé est qu'il met en oeuvre un appareillage encombrant, qu'il est difficile de déplacer pour optimiser son positionnement dans un environnement donné. De plus, il nécessite une phase d'acquisition d'au moins une image, constituant une étape supplémentaire venant allonger la durée du procédé.

Un procédé plus simple consiste pour un porteur de lunettes à se saisir d'un carton troué ou d'un objet équivalent, et à regarder à travers le trou un point identifié. Un optométriste placé en face dudit porteur se focalise sur son regard pour déterminer quel est son oeil directeur. Un tel procédé demeure approximatif et particulièrement contraignant pour le porteur, qui est obligé de se livrer à une manipulation peu aisée du dispositif de visée constitué par le carton troué.

Le document FR-A-2 972 977 divulgue un dispositif de détermination de l'oeil directeur d'un patient comportant une cible visible par le patient en position de mesure, un moyen d'acquisition d'une image du visage du patient en position de mesure, et un masque à positionner entre, d'un côté, le visage du patient en position de mesure, et, de l'autre, la cible et le moyen d'acquisition. Les dispositifs de visée selon l'invention permettent de réaliser un procédé de détermination de l'oeil directeur d'un porteur de lunettes, en s'affranchissant des inconvénients relevés dans l'état de la technique. De cette manière, un procédé de détermination de l'oeil directeur selon l'invention, est réalisé de façon ergonomique, pratique et confortable pour le porteur de lunettes, et peut être mis en oeuvre de façon optimisée dans tout type d'environnement, car il implique un appareillage peu encombrant et simplifié.

L'invention a pour objet un équipement pour la mise en oeuvre d'un procédé de détermination de l'oeil directeur d'un porteur de lunettes, comprenant une cible, un moyen d'occultation d'au moins un oeil, et un dispositif de visée doté d'une fenêtre optique.

La principale caractéristique d'un équipement selon l'invention est que le dispositif de visée comprend un capteur électronique permettant d'identifier un déplacement dudit dispositif de visée, lors de différentes étapes de visualisation de la cible lors dudit procédé. Un tel équipement permet d'avoir directement, grâce au capteur électronique, toutes les informations caractérisant un déplacement du dispositif de visée, comme le sens, l'amplitude et la durée du déplacement. Il est supposé que le capteur électronique est relié avec ou sans fil, à un système intégré ou externe, de réception et de traitement du signal émis par ledit capteur, pour évaluer le sens ainsi que l'amplitude de déplacement du dispositif de visée, lors de la visualisation de la cible à travers la fenêtre optique du dispositif de visée.. Avantageusement le capteur électronique est un capteur 3D. Ce capteur électronique, qui permet de connaitre directement le sens de déplacement et le positionnement du dispositif de visée dans l'espace, peut, par exemple, être de type magnéto-gyroscopique. Avantageusement, ledit capteur est un accéléromètre et/ou un inclinomètre. Le dispositif de visée est un objet que le porteur peut saisir avec ses mains et qu'il peut orienter à sa convenance dans l'espace. Une fenêtre optique est une ouverture limitée, placée dans le dispositif de visée. Cette ouverture peut être simple et de taille constante. Elle peut également être de taille variable, au moyen par exemple, d'un obturateur coulissant.

Optionnellement, cette ouverture peut être matérialisée par un verre, pouvant être coloré ou quadrillé. Une telle ouverture peut être, soit pratiquée directement dans le dispositif de visée, soit ajoutée audit dispositif de visée par l'intermédiaire d'une pièce d'interface rapportée. La cible est un objet identifiable pouvant se fixer contre un mur, sur le sol ou être posée sur une table. Cette cible peut par exemple être constituée par une bande adhésive portant un motif distinctif. Le moyen d'occultation d'au moins un oeil, peut être constitué par un objet venant directement se placer sur le visage d'un porteur, ou par un objet venant se fixer sur une monture de lunettes. Ce moyen d'occultation peut par exemple, être constitué par un cache opaque venant se placer devant un oeil pour l'empêcher de voir. Il peut être de nature mécanique et être géré manuellement. Il peut également être de nature électronique, et être piloté automatiquement par l'intermédiaire d'une télécommande.

Avantageusement, le dispositif de visée est une tablette, le capteur électronique étant intégré à ladite tablette. Cette tablette peut être simplement représentée par un support avec un cache ajouré, ou être de type électronique. Dans cette deuxième catégorie, elle peut être dotée d'un écran tactile. Dans l'hypothèse où cette tablette est d'origine électronique, elle peut soit avoir été spécifiquement conçue pour effectuer des prises de mesure dans le cadre de la définition de montures, soit être constituée par une tablette déjà existante et connue du grand public pour différents usages domestiques, tels que par exemple une connexion internet, des prises de photos et/ou vidéos et divers téléchargements. Selon une autre configuration possible, le capteur électronique peut être extérieur à la tablette et peut venir se fixer à celle-ci.

De façon préférentielle, la cible présente un motif central et des motifs secondaires ordonnés, de sorte que la seule visualisation des motifs secondaires permet de déduire dans quelle direction est située le motif central. De cette manière, un porteur de lunette qui ne peut visualiser que les motifs secondaires en raison de l'occultation de l'un de ses yeux, saura directement dans quel sens déplacer le dispositif de visée pour observer le motif central. Le motif central peut être avantageusement représenté par une figure géométrique simple, de type cercle, carré, losange ou triangle. Les motifs secondaires peuvent par exemple être représentés par des chevrons, des flèches ou des triangles.

Préférentiellement, la cible est pliable et peut se ranger dans un compartiment associé à la tablette. Cette cible peut par exemple être constituée par un carton plastifié ou une bande plastique. Elle peut également présenter une face adhésive. Le fait qu'elle soit pliable lui permet d'occuper un volume réduit et d'être facilement transportée ou rangée.

Avantageusement, le capteur électronique permet d'identifier le sens, l'amplitude, la direction, la durée et les accélérations des déplacements du dispositif de visée.

De façon avantageuse, la tablette est une tablette électronique équipée d'une caméra et d'une fenêtre optique. Cette tablette peut avoir été spécifiquement conçue pour des prises de mesure dans le domaine de l'optique, pour personnaliser une paire de lunettes correctrices, ou être constituée par une tablette développée pour diverses applications comme une connexion internet, l'acquisition d'images. Une caméra additionnelle peut par exemple servir à déterminer une distance de lecture pour un porteur de lunettes. La fenêtre optique peut figurer sur une pièce rapportée et destinée à venir se fixer sur la tablette.

Avantageusement, un équipement selon l'invention, comprend une tablette munie du capteur électronique et un kit fonctionnel apte à être monté sur ladite tablette, ledit kit incluant au moins une pièce support, la fenêtre optique, le moyen d'occultation et la cible, la pièce support étant destinée à être montée sur la tablette pour servir de point de fixation à la fenêtre optique. Ce kit incorpore tous les éléments nécessaires à la détermination de l'oeil directeur. La pièce support vient s'adapter sur la tablette et sert d'embase de fixation à la fenêtre optique. Si le moyen d'occultation est électronique, le kit contient également la télécommande qui sert à actionner ledit moyen d'occultation. Selon un mode de réalisation préféré d'un équipement selon l'invention, la pièce support du kit, est conformée pour servir de sacoche de transport à tous les autres éléments constitutifs dudit kit.

De façon préférentielle, le moyen d'occultation est électronique et fonctionne au moyen d'une télécommande. De cette manière, ledit moyen fonctionne automatiquement en étant commandé à distance, et évite une intervention manuelle délicate de la part d'une tierce personne, pouvant être jugée intrusive par le porteur de lunettes.

Préférentiellement, la tablette est dotée de marqueurs de repérage prévus pour visualiser dans l'espace ladite tablette. Ces marqueurs peuvent par exemple être représentés par des mini damiers comprenant chacun deux cases blanches et deux cases noires. Ces marqueurs inactifs permettent de repérer facilement et nettement la position de la tablette dans l'espace, et notamment sur une image.

De façon avantageuse, le capteur électronique est un inclinomètre et/ou un accéléromètre 3D.

L'invention a pour deuxième objet un procédé de détermination de l'oeil directeur d'un porteur de lunettes au moyen d'un équipement conforme à l'invention, caractérisé en ce qu'il comprend les étapes suivantes :
- Une première étape de visualisation, les deux yeux ouverts, de la cible par le porteur à travers la fenêtre optique du dispositif de visée qu'il tient en main et dont il ajuste la position,
- Une deuxième étape de visualisation de ladite cible avec un seul oeil, sélectionné avec le moyen d'occultation, en déplaçant le dispositif de visée si cela s'avère nécessaire,
- Une troisième étape optionnelle de visualisation de ladite cible avec l'autre oeil sélectionné avec le moyen d'occultation en déplaçant ledit dispositif de visée si cela s'avère nécessaire,
- Une quatrième étape de détermination de l'oeil directeur fondée sur chaque déplacement du dispositif de visée, détecté par le capteur électronique.

Autrement dit, une fois que le porteur visualise la cible les deux yeux ouverts à travers la fenêtre optique, il fige le dispositif de visée dans une première position appropriée. Ensuite un moyen d'occultation permet d'occulter alternativement un oeil puis l'autre afin de mettre en évidence la disparition de la cible observée, et de provoquer si besoin un mouvement de translation de la tablette pour aligner l'oeil avec la cible.

Lors de la deuxième étape, si l'oeil qui n'est pas directeur est occulté, le porteur va pouvoir visualiser la cible avec son oeil directeur à travers la fenêtre optique du dispositif, sans avoir à déplacer de façon significative la position dudit dispositif de visée. La troisième étape de visualisation consiste à occulter l'autre oeil qui est dans ce cas son oeil directeur. Puisqu'il ne voit pas la cible avec son oeil non directeur, le porteur déplace significativement le dispositif de visée pour visualiser la cible avec ledit oeil non directeur.

Si lors de la deuxième étape son oeil directeur est occulté, il ne pourra pas voir la cible avec son oeil non directeur. Il déplace alors significativement le dispositif de visée dans une seconde position pour voir la cible avec son oeil non directeur. Dans la troisième étape l'oeil non directeur du porteur est occulté afin de visualiser la cible avec son oeil directeur. Dans cette situation il ne voit pas la cible avec son oeil directeur, le porteur déplace le dispositif de visée pour visualiser la cible avec ledit oeil directeur.

Les différents déplacements, nombres, amplitudes et formes des signaux du dispositif de visée durant ces étapes, sont enregistrés par le capteur électronique, et le couplage avec l'information d'occultation d'un oeil donné permet de définir l'oeil directeur du porteur. Une analyse statistique permet optionnellement d'affiner le résultat.

Avantageusement l'analyse des signaux permet de fournir un degré de pertinence plus ou moins marquée pour l'oeil directeur déterminé que l'on appelle prédominance oculaire.

Avantageusement des diodes électroluminescentes associées au moyen d'occultation permettent de guider l'opticien durant le procédé de mesures. Par exemple, lorsque la diode clignote en vert le procédé est en cours de mesure. Lorsque celle-ci devient fixe l'oeil directeur est détecté. Enfin, lorsque la diode est orange une erreur est détectée, traduisant soit un mouvement irrégulier ou trop important.

Avantageusement, si lors de la deuxième étape le porteur a été contraint de déplacer le dispositif de visée pour visualiser la cible dans une seconde position, le procédé selon l'invention comprend une première étape intermédiaire comprise entre la deuxième et la troisième étape, ladite première étape intermédiaire consistant à replacer ledit dispositif de visée dans la première position obtenue lors de la première étape, ledit replacement constituant un déplacement qui est détecté par le capteur électronique.

Si lors de la troisième étape le porteur a été contraint de déplacer le dispositif de visée pour visualiser la cible dans une troisième position, le procédé selon l'invention comprend une seconde étape intermédiaire comprise entre la troisième et la quatrième étape, ladite seconde étape intermédiaire consistant à replacer ledit dispositif de visée dans la première position obtenue lors de la première étape, ledit replacement constituant un déplacement qui est détecté par le capteur électronique.

La mesure de l'oeil directeur peut se faire en couplage avec le moyen d'occultation en comptabilisant le nombre de déplacements réalisés par chaque oeil occultés.

De façon préférentielle, la cible présente un motif central et des motifs secondaires ordonnés permettant de déduire dans quelle direction est située le motif central, et lorsque l'oeil directeur est occulté lors de la deuxième ou de la troisième étape, le porteur sait dans quelle direction il doit déplacer le dispositif de visée pour visualiser le motif principal avec son oeil non directeur. Ce type de cible permet d'informer le porteur du sens dans lequel il doit déplacer le dispositif de visée pour visualiser le motif central avec son oeil non directeur, lorsque son oeil directeur est occulté et qu'il ne visualise que les motifs secondaires avec son oeil non directeur.

Préférentiellement, la deuxième et la troisième étape sont précédées chacune d'une étape d'actionnement du moyen d'occultation, ledit moyen d'occultation étant de type électronique et son actionnement s'effectuant à distance au moyen d'une télécommande.

De façon avantageuse, le procédé comprend une étape de présentation des résultats au moyen de l'affichage d'une photo montrant les yeux ouverts du porteur de lunettes ainsi qu'une indication visuelle sur ladite photo de l'oeil directeur. Optionnellement, un indicateur sur le degré de prédominance oculaire de cet oeil directeur peut également être affiché. Cette photo peut apparaitre, soit sur un écran d'ordinateur, soit sur un support papier.

Avantageusement, le procédé comprend une étape de détermination d'un indicateur de prédominance oculaire.

On donne ci-après, une description détaillée, d'un mode de réalisation préféré d'un équipement et d'un procédé de détermination d'un oeil directeur selon l'invention, en se référant aux figures 1 à 6C.
- La figure 1A est une vue de face d'un porteur de lunettes équipé d'un moyen d'occultation manuel d'au moins l'un de ses yeux et appartenant à un équipement selon l'invention, l'oeil gauche dudit porteur étant occulté,
- La figure 1B est une vue de face d'un porteur de lunettes équipé d'un moyen d'occultation manuel d'au moins l'un de ses yeux et appartenant à un équipement selon l'invention, l'oeil droit dudit porteur étant occulté,
- La figure 2 est une vue de face d'un moyen d'occultation électronique d'au moins l'un des yeux du porteur et appartenant à un équipement selon l'invention,
- La figure 3 est une cible d'un équipement selon l'invention,
- La figure 4A est une vue en perspective d'une tablette d'un équipement selon l'invention équipé d'une fenêtre optique,
- La figure 4B est un kit comprenant des éléments d'un équipement selon l'invention,
- Les figures 5A, 5B et 5C représentent des vues de face d'un individu regardant une cible à travers la fenêtre optique d'une tablette, respectivement les deux yeux ouverts, l'oeil droit occulté puis l'oeil gauche occulté, lesdites vues représentant les trois étapes principales d'un procédé selon l'invention,
- La figure 6 illustre un exemple d'enregistrement du signal émis par le capteur 3D, lors d'un procédé selon l'invention.

Un équipement pour la mise en oeuvre d'un procédé de détermination de l'oeil directeur d'un porteur 1 de lunettes, comprend une cible 2, un moyen d'occultation 3,4 d'au moins un oeil dudit porteur 1, et une tablette 5,50 dotée d'une fenêtre optique 6 et d'un capteur électronique 3D de type magnéto-gyroscopique. Ledit capteur est relié à un calculateur intégré à la tablette doté d'un module de traitement du signal, permettant d'évaluer en particulier le sens, l'amplitude, la direction, la durée et les accélérations des déplacements de la tablette 5,50 au cours des différentes étapes d'un procédé de détermination de l'oeil directeur selon l'invention.

Préférentiellement, un seul un axe X du capteur électronique 3D fixé à la tablette 5,50 correspondant à l'axe X de déplacement horizontal de ladite tablette 5,50 est privilégié pour les mesures. Par exemple, une mesure de l'accélération du de cette tablette 5,50 le long de l'axe X horizontal est enregistrée toutes les 41 ms sur une plage allant de -4g à +4g. Les mesures sont signées et permettent ainsi à partir du signal enregistré d'évaluer le sens de déplacement de la tablette 5,50. Afin de supprimer le léger bruit du signal, lié au déplacement naturel et de très faible amplitude du porteur et/ou à la non-horizontalité de la tablette 5,50, avant chaque phase de mesure, la valeur moyenne du signal est déterminée et sauvegardée, puis est soustraite pour la suite des mesures.

Pour chaque mouvement de la tablette 5,50 le calculateur évalue sur le signal les valeurs crête à crête entre les amplitudes minimales et maximales des mouvements, ainsi que la distribution des pics, pour différencier un mouvement significatif de la tablette lié au protocole de mesure de l'oeil directeur, d'une absence de mouvement. Un mouvement très lent est assimilé à une absence de mouvement. Par exemple, une amplitude crête à crête inférieure à une accélération seuil de 0.02 g est considérée comme une absence de mouvement. Un mouvement associé à une accélération supérieure à ce dernier seuil, est considéré comme ayant une accélération significative. Ce seuil peut être ajusté en fonction d'un type de population. En effet, cette valeur seuil sera plus élevée pour une population âgée que pour une population plus jeune. De même, cette valeur seuil sera plus élevée pour une personne active que pour une personne sédentaire.

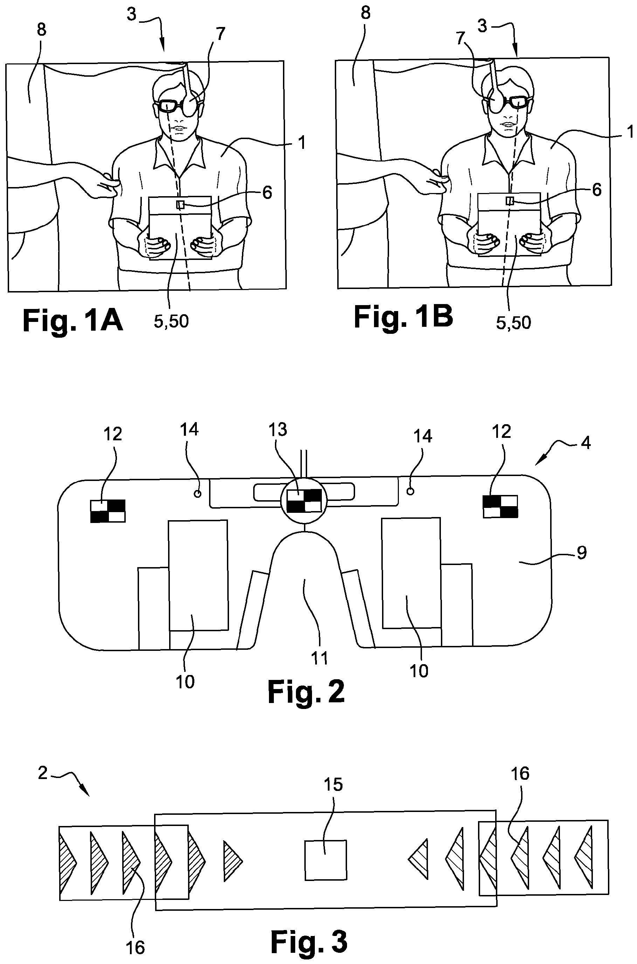

En se référant aux figures 1A et 1B, le moyen d'occultation 3 peut être représenté par deux volets 7 opaques, aptes à venir se placer en surépaisseur sur les verres d'une monture de lunettes. Sur la figure 1A, un opticien 8 a apposé un tel volet 7 sur le verre gauche du porteur 1, afin que seul son oeil droit puisse voir. Sur la figure 1B, l'opticien 8 a placé le volet 7 sur le verre droit pour que seul l'oeil gauche du porteur puisse voir. Généralement, les volets 7 ne sont utilisés que pour occulter un seul oeil à la fois.

En se référant à la figure 2, le moyen d'occultation 4 est de type électronique et peut être commandé automatiquement et à distance, par l'intermédiaire d'une télécommande. Il se présente sous la forme d'un masque 9 destiné à venir recouvrir les deux verres d'une monture, ledit masque 9 étant muni de deux ouvertures pouvant chacune être obturée par un volet 10 opaque, indépendamment l'une de l'autre. La distance séparant les deux ouvertures correspond approximativement à la distance séparant les deux yeux du porteur 1. Ainsi, en actionnant la télécommande, chaque volet 10 peut coulisser de façon autonome dans le masque 9, soit pour obturer une ouverture, soit pour libérer ladite ouverture si elle a été préalablement obturée. Le masque 9 présente une encoche 11 centrale pour permettre le passage du nez du porteur 1, ainsi qu'une série de trois marqueurs 12,13 matérialisés chacun par un mini-damier à quatre cases dont deux sont noires et les deux autres sont blanches. Ces trois marqueurs 12,13 sont alignés selon une direction parallèle à un axe reliant les deux ouvertures, un marqueur 13 étant en position centrale sur le masque 9 au niveau de ladite encoche 11, et les deux autres 12 marqueurs étant en position latérale. Un tel moyen d'occultation 4 peut également comporter au moins une diode 14 électroluminescente de signalisation permettant de renseigner l'opticien sur l'état d'avancée du procédé de détermination de l'oeil directeur selon l'invention.

En se référant à la figure 3 un équipement selon l'invention comporte une cible 2 allongée, sous la forme d'une bande rectangulaire possédant un carré central 15 entouré de triangles 16 pointant vers ledit carré central 15. En effet, de par et d'autre du carré central 15, la cible 2 présente une série de triangles 16 régulièrement espacés, chacun desdits triangles 16 ayant un sommet orienté en direction du carré central 15. Les deux séries de triangles placées autour du carré central peuvent avoir des couleurs différentes. Ces triangles agissent comme des éléments de guidage pour indiquer l'endroit où est positionné le carré central 15. De cette manière, un porteur 1 qui est amené à ne pouvoir visualiser qu'une série de triangles 16, en raison d'un oeil occulté, saura directement dans quel sens il devra déplacer la tablette 5,50 pour visualiser le carré central 16 avec l'autre oeil. La cible 2 est placée par exemple à environ 3m du porteur 1, soit au sol, soit sur une table.

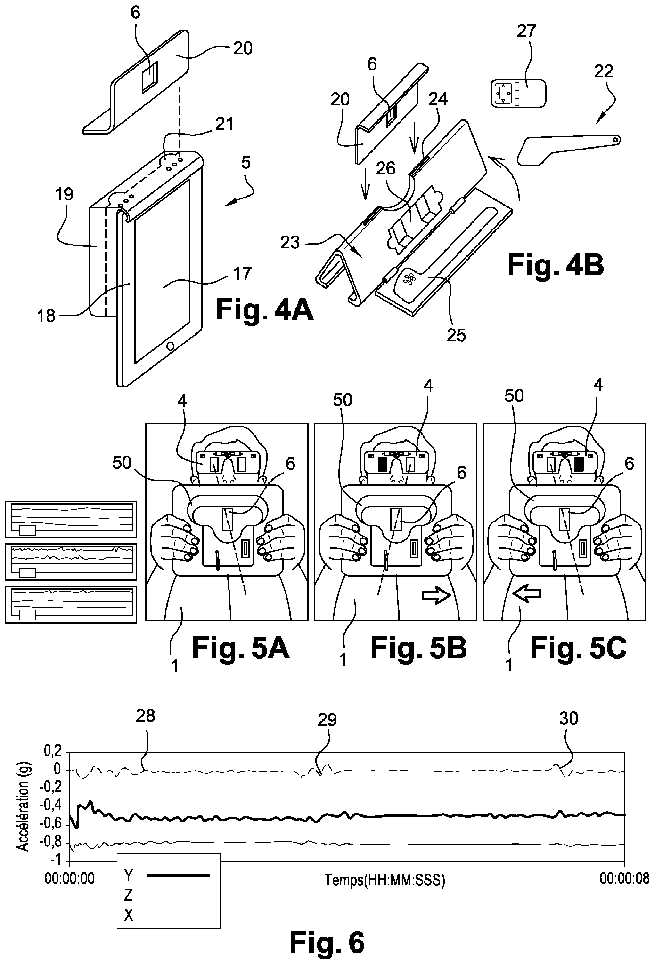

En se référant à la figure 4A, la tablette 5 peut être une tablette électronique déjà existante et connue du grand public, et qui a été développée notamment pour avoir accès à internet, pour pouvoir prendre des photos ou vidéos et pour procéder à divers téléchargements. Ces tablettes 5 sont généralement de faible épaisseur et possèdent un écran tactile 17 entouré par un cadre 18 rectangulaire. Elles comportent déjà un capteur électronique 3D intégré, mais sont dépourvues de fenêtre optique 6. Il faut souligner qu'une fenêtre optique 6 se caractérise par une ouverture limitée, pouvant ou non être occupé par un verre transparent, et à travers de laquelle le porteur 1 va pouvoir regarder la cible 2. Pour ce type de tablette 5, un équipement selon l'invention prévoit une pièce de réception 19 ainsi qu'une pièce de visée 20 munie de la fenêtre optique 6 et apte à s'insérer dans ladite pièce de réception 19. La pièce de réception 19, qui comporte une fente 21, vient se poser facilement et rapidement sur un bord de la tablette 5, puis la pièce de visée 20 est ensuite insérée dans ladite fente 21 de manière à faire émerger la fenêtre optique 6 de ladite tablette 5.

En se référant à la figure 4B, l'équipement selon l'invention, prévoit un kit 22 de montage complet sur une tablette 5 électronique déjà existante pour réaliser un procédé de détermination de l'oeil directeur selon l'invention. Ce kit 22 de montage possède une pièce support centrale 23 et compartimentée, dont un logement 24 est prévu pour la pièce de visée 20 munie de la fenêtre optique 6, dont un autre logement 25 est prévu pour le moyen d'occultation 4 électronique, et dont un autre logement 26 est prévu pour la télécommande 27 dudit moyen d'occultation 4. Un compartiment supplémentaire, non visible sur la figure, est réservé à la cible pliable 2.

En se référant aux figures 5A à 5C, selon un deuxième mode de réalisation préféré d'un équipement selon l'invention, la tablette 50 peut être constituée par une tablette analogue à celle qui est, par exemple, décrite dans la demande de brevet FR2972339. Cette tablette 50 possède une fenêtre optique 6 intégrée et peut supporter un texte de lecture. Des marqueurs à damier positionnés au recto de la tablette 50 permettent de repérer la position de ladite tablette dans l'espace, et donc sur une image. Une telle tablette 50 permet, outre la détermination de l'oeil directeur du porteur 1, l'évaluation d'une distance de lecture au moyen d'un système d'acquisition d'images extérieur.

En se référant aux figures 5A à 5C, un procédé de détermination de l'oeil directeur d'un porteur de lunettes au moyen d'un équipement conforme à l'invention comprend les étapes suivantes :
- Une première étape de visualisation, les deux yeux ouverts, de la cible 2 par le porteur 1 à travers la fenêtre optique 6 de la tablette 5,50 qu'il tient en main et dont il ajuste la position, conformément à ce que montre la figure 5A. Une fois que le porteur 1 visualise la cible 2, on considère que la tablette 5,50 occupe une première position de référence. Cette première position de référence est déterminée à partir de l'oeil directeur du porteur non occulté à cette étape.
- Une deuxième étape de visualisation de ladite cible 2 uniquement par exemple avec son oeil gauche en réglant le moyen d'occultation 3,4 de manière à ce qu'il obture l'oeil droit, à partir de la première position de référence. Si le porteur 1 possède un oeil directeur droit, alors il devra déplacer latéralement la tablette 5,50 jusqu'à une seconde position afin de visualiser le carré central 15 de la cible 2 avec son oeil gauche. Le capteur 3D va alors détecter une première accélération latérale de ladite tablette 5,50. Pour aider le porteur à trouver plus facilement la position de la cible 2, le sens de déplacement de la tablette 5,50 sera directement indiqué par les triangles 16 de la cible 2 entourant le carré central 15, que le porteur 1 visualise avec son oeil gauche. Si le porteur 1 possède un oeil directeur gauche, il pourra alors visualiser directement le carré central 15 de la cible 2 au cours de cette étape, sans avoir à déplacer la tablette 5,50. Le capteur 3D n'enregistre dans ce cas, aucun signal témoignant d'une accélération significative de ladite tablette 5,50.
- Une troisième étape de visualisation de ladite cible 2 uniquement avec son oeil droit, en réglant le moyen d'occultation 3,4 de manière à ce qu'il obture l'oeil gauche. Si le porteur possède un oeil directeur droit, il va devoir repositionner la tablette 5,50 qu'il a précédemment déplacé lors de la deuxième étape jusqu'à une troisième position correspondant à une visualisation du carré central 15 de la cible 2 les deux yeux ouverts. Le capteur 3D va alors enregistrer une accélération correspondant au repositionnement de la tablette 5,50. Si le porteur possède un oeil directeur gauche, il va déplacer latéralement la tablette 5,50 afin de pouvoir visualiser le centre 15 de la cible 2 avec son oeil non directeur. Le capteur 3D va alors enregistrer une accélération liée au déplacement de ladite tablette 5,50. La troisième position peut correspondre sensiblement à la première position.

En se référant à la figure 6, avec un procédé se déroulant suivant le protocole précédent, si le porteur 1 possède un oeil droit directeur, le capteur 3D va enregistrer deux déplacements horizontaux suivant X de la tablette 5,50, l'un pour pouvoir observer le centre 15 de la cible 2 avec son oeil gauche correspondant au déplacement de la tablette de la première position vers la deuxième position, et l'autre pour positionner la tablette 5,50 de la deuxième positon vers la troisième position. S'il possède un oeil gauche directeur, le capteur 3D ne va enregistrer qu'un seul déplacement de tablette 5,50. La courbe 28 représentative d'un mouvement de la tablette 5,50 suivant un axe X horizontal, montre bien deux pics 29,30 correspondant aux deux déplacements de la tablette 5,50, qui ont été nécessaires si le porteur possède un oeil droit directeur. Pour confirmer le diagnostic, la séquence des trois étapes du procédé peut se répéter plusieurs fois dans le temps. Il faut souligner que l'axe Y correspond à un axe vertical et l'axe Z est un axe de profondeur correspondant à l'éloignement ou au rapprochement de la tablette par rapport à l'individu qui est sensiblement orthogonal à l'axe X et Y et que suivant ces deux axes le déplacement de la tablette 5,50 est négligeable.

Avantageusement, un système simplifié peut-être obtenu avec une table prédéterminée, qui est programmée dans le calculateur et qui permet en fonction de l'oeil occulté lors de la première étape, de déterminer uniquement en fonction de la présence ou non d'un pic d'accélération lors de la deuxième étape, quel est l'oeil directeur. Ainsi la connaissance de l'oeil occulté lors de la première étape, se fait par un moyen déclaratif manuel, de type saisie ou acquisition d'images synchronisée suivi d'un traitement d'images, ou si l'on dispose d'un système automatique de détermination de l'oeil occulté, avec l'aide d'un système électronique occultant.

Par exemple, si l'oeil droit est occulté et que l'on trouve un pic significatif dans la seconde étape alors l'oeil directeur est l'oeil droit. Si on ne trouve pas de pic significatif, l'oeil directeur est l'oeil gauche. Et inversement, si l'oeil gauche est occulté dans la première étape et que l'on trouve un pic significatif pour la deuxième étape, l'oeil directeur est l'oeil gauche, sinon c'est l'oeil droit qui est l'oeil directeur. Le pic est considéré comme étant significatif, s'il est supérieur à un seuil prédéfini, par exemple, positionné pour une amplitude crête à crête du pic correspondant à une accélération de 0.02 g.

Cependant, les mesures réalisées dans la troisième phase sont préférables et vont permettre d'affiner le résultat, par la détection de défauts et la vérification de la cohérence des données.
Lors d'un cycle de mesure de l'oeil dominant, sont capturées et sauvegardées plusieurs informations concernant les 2 parties de la mesure, l'une correspondant à l'oeil droit et l'autre à l'oeil gauche:
- Le maximum de l'accélération positive,
- Le minimum de l'accélération négative,
- La distribution des pics de mouvement représentée dans un histogramme.

La distribution des pics correspond à un classement par paliers des échantillons d'intensité. Par exemple nous considérons 11 paliers pour caractériser le signal, avec chaque palier correspondant à une accélération de 0.01g chacun pour les 10 premiers paliers (0->10, 11->20, 21->30, ....) et pour le 11^{ème} palier des valeurs supérieures à 100 mg.

Les deux premiers paliers correspondent aux micromouvements ou absence de mouvements dus aux tremblements de la personne, et représentent une accélération inférieure à 0.02 g. Cette absence de mouvement sera représentée par un grand nombre d'échantillons dans le premier palier, peu dans le second palier, et aucun dans les suivants.

Un mouvement fait à vitesse normale génère typiquement quelques pics dans les paliers supérieurs ou égaux à trois, et beaucoup d'échantillons dans les deux premiers paliers d'intensités.

Après la phase d'acquisition des mouvements, une analyse est faite sur les différentes parties du signal afin de déterminer si la mesure est valide ou si elle doit être rejetée due à un mouvement irrégulier ou bien caractérisé par un bruit important dans la mesure ou encore une absence de mouvement.

Pour cela différents « types de bruit » sont identifiés dans uine phase préalable de calibration et dans laquelle les seuils sont déterminés en fonction des conditions d'utilisations et de l'individu. Dans ces cas, le mouvement est considéré comme incorrect et une alerte est présentée à l'opérateur pour éventuellement recommencer le procédé.

Si les paliers intermédiaires trois et quatre contiennent par exemple chacun plus de 5 échantillons ceci est considéré comme trop de mouvement dans des valeurs de paliers faibles, et est donc assimilé à un tremblement modéré.

Si les six derniers paliers de la distribution contiennent en cumulé plus de 5 échantillons alors ceci est considéré comme trop de mouvements dans les paliers haut et est assimilé à un mouvement anormal, comme par exemple un mouvement trop saccadé ou sans stabilisation sur l'oeil dominant.

Si le signal est considéré valide, alors le calcul de l'oeil directeur, cherche à détecter un mouvement dans chaque partie de la mesure correspondant à la deuxième et troisième étape du procédé.

Un indicateur de prédominance oculaire correspondant à un degré de pertinence plus ou moins marquée de l'oeil directeur déterminé est présenté. Cet indicateur fournit une aide supplémentaire à l'opticien pour caractériser l'oeil directeur de l'individu. La prédominance oculaire peut être marquée, faible ou normale. Cet indicateur est évalué sur la base d'un cycle correspondant à la deuxième et à la troisième étape.

La valeur analogique de dominance est par exemple caractérisée par l'analyse temporelle des signaux et obtenue par le ratio D = TS/ (TR + TS) avec
- TR : moyenne du temps de réaction suite à l'occultation
- TS : moyenne du temps de stabilisation après déplacement.

Les valeurs typiques sont 0,8 à 1,6 s pour TR et 0,8 à 2s pour TS. Ces valeurs sont ajustées classiquement par une courbe d'expérience et par apprentissage

| | **intensité (IS)** | **ratio (RS)** | **réaction (TR)** | **stabilisation (TS)** |
|---|---|---|---|---|
| | mg | signal 1 / s2 | seconde | seconde |
| **valeurs typiques** | | | | |
| élevées | > 150 | >4 | < 0,8 | < 0,8 |
| normales | 100-150 | 3 | 0,8-1,6 | 0,8-2 |
| faibles | 60-100 | 2 | >1,6 | > 2 |
| | | | | |
| **Dominance** | D = IS*(1+RS/10)/ (TR+TS)/2 | | | |
| faible | < | 70 | | |
| normale | > 70 | < 250 | | |
| marquée | > | 260 | | |

Ainsi, si le ratio est inférieur à 30% l'indicateur de prédominance sera faible, s'il est compris entre 30 et 60% il sera considéré comme normale, et il sera considéré comme marqué pour un ratio supérieur à 60%.

Un procédé selon l'invention comprend une quatrième étape de présentation des résultats au moyen de l'affichage d'une photo, montrant les yeux ouverts du porteur 1 de lunettes, ainsi qu'une indication visuelle sur ladite photo permettant d'identifier l'oeil directeur.

Optionnellement, la diode électroluminescente 14 du moyen d'occultation 4 électronique, va pouvoir guider un opticien durant le procédé. Par exemple,

Lorsque celle-ci est verte clignotant, la mesure est en cours et l'oeil directeur n'est pas encore détecté

Lorsque celle-ci devient vert fixe, l'oeil dominant est déterminé

Lorsqu'elle est orange clignotant, l'initialisation de la mesure est en cours

Enfin, lorsqu'elle est orange fixe, la mesure est réalisée.

## Revendications

1. Equipement pour la mise en oeuvre d'un procédé de détermination de l'oeil directeur d'un porteur (1) de lunettes, comprenant une cible (2), un moyen d'occultation (3,4) d'au moins un oeil, et un dispositif de visée (5,50) doté d'une fenêtre optique (6), **caractérisé en ce que** le dispositif de visée (5,50) comprend un capteur électronique permettant d'identifier un déplacement dudit dispositif de visée (5,50), lors de différentes étapes de visualisation de la cible (2) lors dudit procédé.

2. Equipement selon la revendication 1, **caractérisé en ce que** le dispositif de visée est une tablette (5,50), et **en ce que** le capteur électronique est intégré à ladite tablette (5,50).

3. Equipement selon la revendication 2, **caractérisé en ce que** la cible (2) présente un motif central (15) et des motifs secondaires (16) ordonnés, de sorte que la seule visualisation des motifs secondaires (16) permet de déduire dans quelle direction est située le motif central (15).

4. Equipement selon l'une des revendications précédentes **caractérisé en ce que** ledit capteur électronique permet d'identifier le sens, l'amplitude, la direction, la durée et les accélérations des déplacements du dispositif de visée (5,50).

5. Equipement selon l'une quelconque des revendications 2 à 4, **caractérisé en ce que** la tablette est une tablette (5) électronique équipée d'une caméra et d'une fenêtre optique (6).

6. Equipement selon l'une quelconque des revendications 2 à 5, **caractérisé en ce qu'**il comprend une tablette (5,50) munie du capteur électronique et un kit fonctionnel apte à être monté sur ladite tablette (5,50), et **en ce que** ledit kit inclut au moins une pièce support (19), la fenêtre optique (6,20), le moyen d'occultation (3,4) et la cible (2), la pièce support (19) étant destinée à être montée sur la tablette (5,50) pour servir de point de fixation à la fenêtre optique (6,20).

7. Equipement selon l'une quelconque des revendications 1 à 6, **caractérisé en ce que** le moyen d'occultation (4) est électronique et fonctionne au moyen d'une télécommande (27).

8. Equipement selon l'une quelconque des revendications 1 à 7, **caractérisé en ce que** la tablette (5,50) est dotée de marqueurs de repérage prévus pour visualiser dans l'espace ladite tablette (5,50).

9. Equipement selon l'une quelconque des revendications 1 à 8, **caractérisé en ce que** le capteur électronique est un inclinomètre et/ou un accéléromètre 3D.

10. Procédé de détermination de l'oeil directeur d'un porteur de lunettes au moyen d'un équipement conforme à l'une quelconque des revendications 1 à 9, **caractérisé en ce qu'**il comprend les étapes suivantes :
- Une première étape de visualisation, les deux yeux ouverts, de la cible (2) par le porteur (1) à travers la fenêtre optique (6) du dispositif de visée (5,50) qu'il tient en main et dont il ajuste la position,
- Une deuxième étape de visualisation de ladite cible (2) avec un seul oeil, sélectionné avec le moyen d'occultation (3,4), en déplaçant le dispositif de visée (5,50) si cela s'avère nécessaire,
- Une troisième étape optionnelle de visualisation de ladite cible (2) avec l'autre oeil sélectionné avec le moyen d'occultation (3,4), en déplaçant ledit dispositif de visée (5,50) si cela s'avère nécessaire,
- Une quatrième étape de détermination de l'oeil directeur fondée sur chaque déplacement du dispositif de visée (5,50), détecté par le capteur électronique.

11. Procédé selon la revendication 10, **caractérisé en ce que**, si lors de la deuxième étape le porteur (1) a été contraint de déplacer le dispositif de visée (5,50) pour visualiser la cible (2) dans une seconde position, ledit procédé comprend une première étape intermédiaire comprise entre la deuxième et la troisième étape, ladite première étape intermédiaire consistant à replacer ledit dispositif de visée (5,50) dans la première position obtenue lors de la première étape, ledit replacement constituant un déplacement qui est détecté par le capteur électronique.

12. Procédé selon la revendication 11, **caractérisé en ce que** la cible (2) présente un motif central (15) et des motifs secondaires (16) ordonnés permettant de déduire dans quelle direction est située le motif central (15), et **en ce que** lorsque l'oeil directeur est occulté lors de la deuxième ou de la troisième étape le porteur (1) sait dans quelle direction il doit déplacer le dispositif de visée (5,50) pour visualiser le motif central (15) avec son oeil non directeur.

13. Procédé selon l'une quelconque des revendications 10 à 12, **caractérisé en ce que** la deuxième et la troisième étape sont précédées chacune d'une étape d'actionnement du moyen d'occultation (3,4), et **en ce que** ledit moyen d'occultation (4) est de type électronique, son actionnement s'effectuant à distance au moyen d'une télécommande (27).

14. Procédé selon l'une quelconque des revendications 10 à 13, **caractérisé en ce qu'**il comprend une étape de présentation des résultats au moyen de l'affichage d'une photo montrant les yeux ouverts du porteur (1) de lunettes ainsi qu'une indication visuelle sur ladite photo de l'oeil directeur.

15. Procédé selon l'une quelconque des revendications 10 à 14, **caractérisé en ce qu'**il comprend une étape de détermination d'un indicateur de prédominance oculaire.

## Patentansprüche

1. Ausrüstung zur Durchführung eines Verfahrens zur Bestimmung des dominanten Auges eines Brillenträgers (1), umfassend ein Ziel (2), ein Abdeckmittel (3, 4) mindestens eines Auges und eine Visiervorrichtung (5, 50), die mit einem optischen Fenster (6) versehen ist,
**dadurch gekennzeichnet, dass** die Visiervorrichtung (5, 50) einen elektronischen Sensor umfasst, der es gestattet, eine Verschiebung der Visiervorrichtung (5, 50) bei verschiedenen Schritten einer Visualisierung des Ziels (2) während des Verfahrens zu identifizieren.

2. Ausrüstung nach Anspruch 1,
**dadurch gekennzeichnet, dass** die Visiervorrichtung eine Tafel (5, 50) ist, und dadurch, dass der elektronische Sensor in die Tafel (5, 50) integriert ist.

3. Ausrüstung nach Anspruch 2,
**dadurch gekennzeichnet, dass** das Ziel (2) ein zentrales Motiv (15) und geordnete sekundäre Motive (16) derart aufweist, dass die alleinige Visualisierung der sekundären Motive (16) es gestattet abzuleiten, in welcher Richtung das zentrale Motiv (15) angeordnet ist.

4. Ausrüstung nach einem der vorhergehenden Ansprüche,
**dadurch gekennzeichnet, dass** der elektronische Sensor es gestattet, die Richtung, die Amplitude, die Führung, die Dauer und die Beschleunigungen der Verschiebungen der Visiervorrichtung (5, 50) zu identifizieren.

5. Ausrüstung nach einem der Ansprüche 2 bis 4,
**dadurch gekennzeichnet, dass** die Tafel eine elektronische Tafel (5) ist, die mit einer Kamera und einem optischen Fenster (6) ausgestattet ist.

6. Ausrüstung nach einem der Ansprüche 2 bis 5,
**dadurch gekennzeichnet, dass** diese eine Tafel (5, 50) umfasst, die mit einem elektronischen Sensor und einem funktionellen Kit versehen ist, das geeignet ist, auf der Tafel (5, 50) montiert zu werden, und dadurch, dass das Kit mindestens ein Stützstück (19), das optische Fenster (6, 20), das Abdeckmittel (3, 4) und das Ziel (2) aufweist, wobei das Stützstück (19) dazu bestimmt ist, auf der Tafel (5, 50) montiert zu werden, um als Befestigungspunkt für das optische Fenster (6, 20) zu dienen.

7. Ausrüstung nach einem der Ansprüche 1 bis 6,
**dadurch gekennzeichnet, dass** das Abdeckmittel (4) elektronisch ist und mittels einer Fernbedienung (27) funktioniert.

8. Ausrüstung nach einem der Ansprüche 1 bis 7,
**dadurch gekennzeichnet, dass** die Tafel (5, 50) mit Markierungselementen versehen ist, die bereitgestellt sind, um die Tafel (5, 50) im Raum zu visualisieren.

9. Ausrüstung nach einem der Ansprüche 1 bis 8,
**dadurch gekennzeichnet, dass** der elektronische Sensor ein Neigungsmesser und/oder ein Beschleunigungsmesser in 3D ist.

10. Verfahren zur Bestimmung des dominanten Auges eines Brillenträgers mittels einer Ausrüstung nach einem der Ansprüche 1 bis 9,
**dadurch gekennzeichnet, dass** dieses die folgenden Schritte umfasst:
- einen ersten Schritt der Visualisierung, wobei beide Augen offen sind, des Ziels (2) durch den Träger (1) quer durch das optische Fenster (6) der Visiervorrichtung (5, 50), die er in den Händen hält und deren Position er einstellt,
- einen zweiten Schritt der Visualisierung des Ziels (2) mit einem Auge, das mit dem Abdeckmittel (3, 4) ausgewählt wird, indem die Visiervorrichtung (5, 50) verschoben wird, wenn sich dies als notwendig erweist,
- einen dritten optionalen Schritt der Visualisierung des Ziels (2) mit dem anderen Auge, das mit dem Abdeckmittel (3, 4) ausgewählt wird, indem die Visiervorrichtung (5, 50) verschoben wird, wenn sich dies als notwendig erweist,
- einen vierten Schritt der Bestimmung des dominanten Auges basierend auf jeder Verschiebung der Visiervorrichtung (5, 50), die von dem elektronischen Sensor detektiert wird.

11. Verfahren nach Anspruch 10,
**dadurch gekennzeichnet, dass**, wenn beim zweiten Schritt der Träger (1) dazu gebracht wurde, die Visiervorrichtung (5, 50) zu verschieben, um das Ziel (2) in einer zweiten Position zu visualisieren, das Verfahren einen ersten Zwischenschritt zwischen dem zweiten und dem dritten Schritt umfasst, wobei der erste Zwischenschritt darin besteht, die Visiervorrichtung (5, 50) in der ersten Position, die während des ersten Schritts erhalten wird, neu zu platzieren, wobei das Neuplatzieren eine Verschiebung darstellt, die von dem elektronischen Sensor detektiert wird.

12. Verfahren nach Anspruch 11,
**dadurch gekennzeichnet, dass** das Ziel (2) ein zentrales Motiv (15) und geordnete sekundäre Motive (16) aufweist, die es gestatten abzuleiten, in welcher Richtung das zentrale Motiv (15) angeordnet ist, und dadurch, dass, wenn das dominante Auge beim zweiten oder beim dritten Schritt abgedeckt wird, der Träger (1) weiß, in welcher Richtung er die Visiervorrichtung (5, 50) verschieben muss, um das zentrale Motiv (15) mit seinem nicht dominanten Auge zu visualisieren.

13. Verfahren nach einem der Ansprüche 10 bis 12,
**dadurch gekennzeichnet, dass** dem zweiten und dem dritten Schritt jeweils ein Betätigungsschritt des Abdeckmittels (3, 4) vorausgeht, und dadurch, dass das Abdeckmittel (4) vom elektronischen Typ ist, wobei seine Betätigung aus der Entfernung mittels einer Fernbedienung (27) erfolgt.

14. Verfahren nach einem der Ansprüche 10 bis 13, **dadurch gekennzeichnet, dass** dieses einen Schritt der Präsentation der Ergebnisse mittels einer Anzeige eines Fotos umfasst, das die offenen Augen des Brillenträgers (1) sowie eine visuelle Darstellung des dominanten Auges auf dem Foto zeigt.

15. Verfahren nach einem der Ansprüche 10 bis 14,
**dadurch gekennzeichnet, dass** dieses einen Schritt der Bestimmung eines Indikators der Augendominanz umfasst.

## Claims

1. Apparatus for implementing a method for determining the dominant eye of a spectacle wearer (1), comprising a target (2), a means (3, 4) for occulting at least one eye, and a sighting device (5, 50) equipped with an optical window (6), **characterized in that** the sighting device (5, 50) comprises an electronic sensor making it possible to identify a movement of said sighting device (5, 50), during said method during various steps of viewing the target (2).

2. Apparatus according to Claim 1, **characterized in that** the sighting device is a tablet (5, 50), and **in that** the electronic sensor is integrated into said tablet (5, 50).

3. Apparatus according to Claim 2, **characterized in that** the target (2) has a central pattern (15) and secondary patterns (16) ordered so that viewing of the secondary patterns (16) alone makes it possible to deduce in which direction the central pattern (15) is located.

4. Apparatus according to one of the preceding claims, **characterized in that** said electronic sensor makes it possible to identify the sign, amplitude, direction, duration and the accelerations of the movements of the sighting device (5, 50).

5. Apparatus according to any one of Claims 2 to 4, **characterized in that** the tablet is an electronic tablet (5) equipped with a camera and an optical window (6) .

6. Apparatus according to any one of Claims 2 to 5, **characterized in that** it comprises a tablet (5, 50) equipped with the electronic sensor and an operational kit able to be mounted on said tablet (5, 50), and **in that** said kit includes at least one holding part (19), the optical window (6, 20), the occulting means (3, 4) and the target (2), the holding part (19) being intended to be mounted on the tablet (5, 50) in order to serve as a point for fastening the optical window (6, 20).

7. Apparatus according to any one of Claims 1 to 6, **characterized in that** the occulting means (4) is electronic and is operated by means of a remote control (27) .

8. Apparatus according to any one of Claims 1 to 7, **characterized in that** the tablet (5, 50) is equipped with locating markers provided for viewing said tablet (5, 50) in space.

9. Apparatus according to any one of Claims 1 to 8, **characterized in that** the electronic sensor is an inclinometer and/or a 3D accelerometer.

10. Method for determining the dominant eye of a spectacle wearer by means of an apparatus according to any one of Claims 1 to 9, **characterized in that** it comprises the following steps:
- A first step in which the wearer (1) views, with both eyes open, the target (2) through the optical window (6) of the sighting device (5, 50), which he holds in his hand and the position of which he adjusts;
- A second step of viewing said target (2) with a single eye, selected with the occulting means (3, 4), the wearer (1) moving the sighting device (5, 50) if it proves to be necessary;
- An optional third step of viewing said target (2) with the other eye, selected with the occulting means (3, 4), the wearer (1) moving said sighting device (5, 50) if it proves to be necessary; and
- A fourth step of determining the dominant eye based on each movement of the sighting device (5, 50) detected by the electronic sensor.

11. Method according to Claim 10, **characterized in that**, if in the second step the wearer (1) needed to move the sighting device (5, 50) in order to view the target (2) in a second position, said method comprises a first intermediate step comprised between the second and third steps, said first intermediate step consisting in repositioning said sighting device (5, 50) in the first position obtained in the first step, said repositioning constituting a movement that is detected by the electronic sensor.

12. Method according to Claim 11, **characterized in that** the target (2) has a central pattern (15) and ordered secondary patterns (16) making it possible to deduce in which direction the central pattern (15) is located, and **in that** when the dominant eye is occulted in the second or third step the wearer (1) knows in which direction he must move the sighting device (5, 50) in order to view the central pattern (15) with his non-dominant eye.

13. Method according to any one of Claims 10 to 12, **characterized in that** the second and third steps are each preceded by a step of actuating the occulting means (3, 4), and **in that** said occulting means (4) is electronic and actuated remotely by means of a remote control (27).

14. Method according to any one of Claims 10 to 13, **characterized in that** it comprises a step of presenting results by means of display of a photo showing the open eyes of the spectacle wearer (1) and a visual indication in said photo of the dominant eye.

15. Method according to any one of Claims 10 to 14, **characterized in that** it comprises a step of determining an ocular predominance indicator.
